(19) Europäisches Patentamt
European Patent Office
Office européen des brevets

(11) Publication number: **0 226 642 B1**

(12) # EUROPEAN PATENT SPECIFICATION

(45) Date of publication of patent specification: **02.05.90**

(51) Int. Cl.$^5$: **C 07 C 275/54,** C 07 C 335/26, A 01 N 47/34

(21) Application number: **86903574.1**

(22) Date of filing: **30.05.86**

(86) International application number: **PCT/JP86/00272**

(87) International publication number: **WO 86/07355 18.12.86 Gazette 86/27**

(54) **BENZOYLUREA DERIVATIVES, PROCESS FOR THEIR PREPARATION, AND INSECTICIDES.**

(30) Priority: **04.06.85 JP 119851/85**

(43) Date of publication of application: **01.07.87 Bulletin 87/27**

(45) Publication of the grant of the patent: **02.05.90 Bulletin 90/18**

(84) Designated Contracting States: **CH DE FR GB IT LI NL**

(56) References cited:
**JP-A-5 289 646**
**JP-A-5 615 259**
**JP-A-59 106 454**
**JP-A-60 246 359**

**No relevant documents have been disclosed.**

(73) Proprietor: **NIPPON SODA CO., LTD.**
**2-1, Ohtemachi 2-chome**
**Chiyoda-ku, Tokyo 100 (JP)**

(72) Inventor: **NAGASAKI, Fumihiko 680, Takada**
**Odawara-shi**
**Kanagawa 250-02 (JP)**
Inventor: **HAGIWARA, Kenji 16-5, Nakazato**
**Odawara-shi**
**Kanagawa 250 (JP)**
Inventor: **HARA, Tamio 2-6-23, Koma**
**Ohiso-machi, Naka-gun**
**Kanagawa 255 (JP)**
Inventor: **YAMADA, Tomio 28-3, Kurobeoka**
**Hiratsuka-shi**
**Kanagawa 254 (JP)**
Inventor: **MATSUDA, Michihiko 5-14-12-253,**
**Ohgi-machi**
**Odawara-shi**
**Kanagawa 250 (JP)**
Inventor: **TAKAHASHI, Hidemitsu 420-2,**
**Nagamochi**
**Hiratsuka-shi**
**Kanagawa 259-12 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 226 642 B1

(74) Representative: **van der Beek, George Frans, Ir. et al**
**Nederlandsch Octrooibureau Scheveningseweg 82 P.O. Box 29720**
**NL-2502 LS 's-Gravenhage (NL)**

**Description**

This Invention concerns new benzoylurea derivatives, the processes of their production and their uses as insecticides.

It is widely known that N-benzoyl-N'-phenylurea derivatives have an insecticidal action. Typical of these derivatives on the market is N-2,6-difluorobenzoyl-N'-4-chlorophenylurea (diflubenzuron), which is disclosed in NL—A—7007040. The following are also known as compounds which like those covered by this Invention have fluorine atom at the 2-position of the aniline portion.

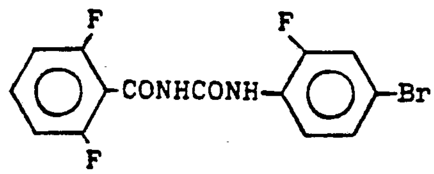

(EP-88343)

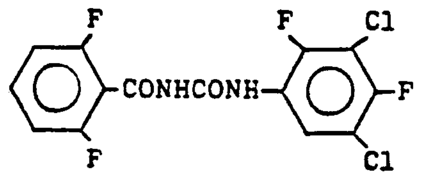

(EP-52833)

Further, in the US—4139636 or EP—71279, the following are also disclosed as compounds having haloalkoxy radical at the 4-position of the aniline portion.

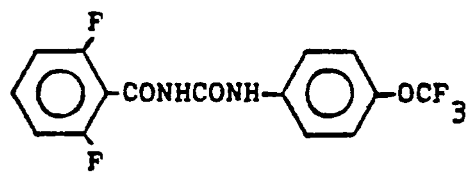

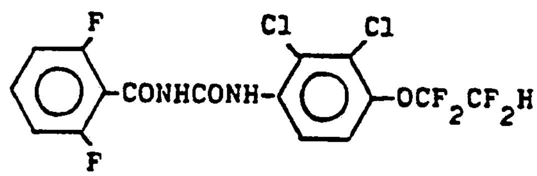

The compounds which this Invention covers may be included in the scope of the above patent claims, but in the specification, nothing is specified regarding the compounds which have fluorine atoms, as halogen atom of substituents for the aniline portion.

The inventors et al. have assiduously conducted a range of research in efforts to find compounds which have a wider insecticidal spectrum and superior larvicidal and ovicidal actions than the known compounds mentioned above.

As a result of the research which the inventors et al. conducted for the above purpose, they have found that benzoylurea derivatives that have 2-fluoro-4-trifluoromethoxy radical as substituents of the aniline portion, do excel the above-mentioned known compounds, or those compounds below which can easily be considered from the aforesaid known literature,

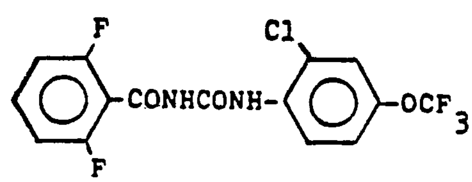

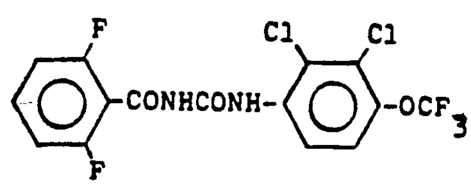

not only in insecticidal effect but in ovicidal action as well. Fruits moth, leaf miner or other insect pests, after they hatched infiltrate and injure fruits or leaves, and to compact them, the emergence of preventive chemicals with ovicidal action has long been awaited. In this sense, also, the compounds which this Invention covers present the type of insecticides which offers a wide range of uses. Hence this Invention concerns the compound expressed by the general formula below:

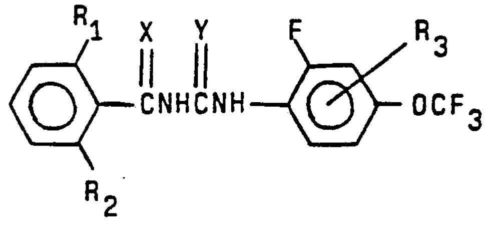

(wherein $R_1$ denotes halogen atom or methyl radical, $R_2$ denotes hydrogen atom or halogen atom, $R_3$ denotes hydrogen atom, halogen atom or methyl radical, X and Y denote oxygen atom or sulfur atom), the processes of their production, and the insecticide which contain said compound as active ingredients. Of these compounds covered by this Invention, those whose $R_1$, $R_2$ and $R_3$ are compounds of fluorine atom, are particularly strong in insecticidal and ovicidal action.

The compound of this Invention can be manufactured in the method expressed by the reaction formulas below.

A. X : Oxygen atom

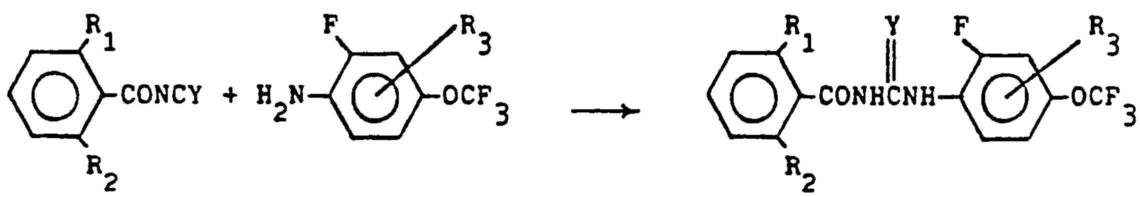

B.

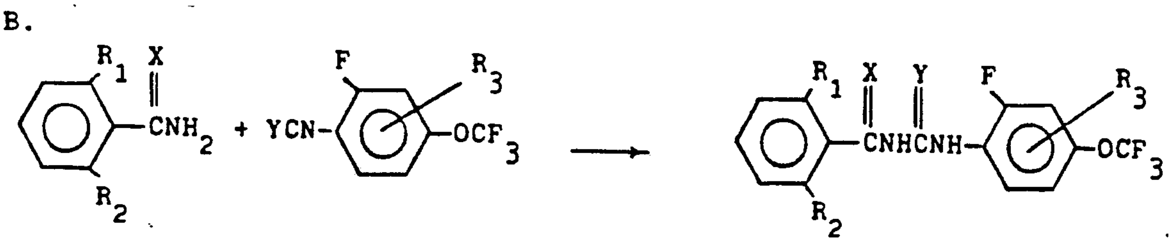

C. X : Sulfur atom

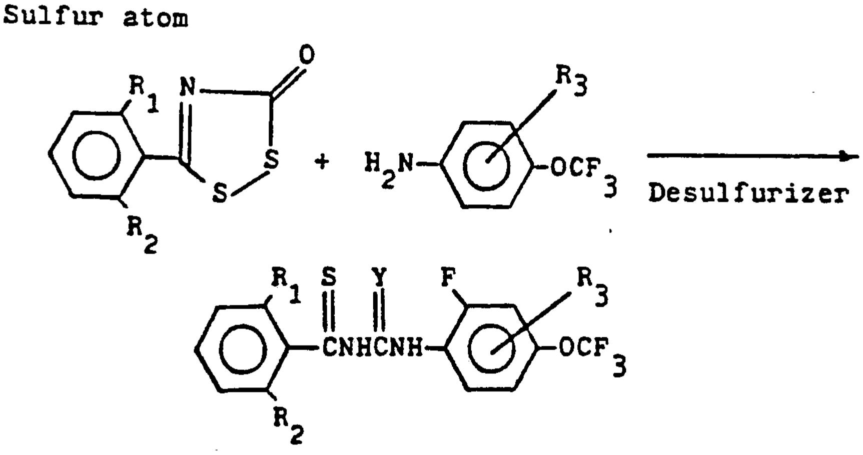


The reactions under the reaction formulas A or B above are carried out in an organic solvent for 30 minutes to 1 hour, at a reaction temperature ranging from 0 to 80°C. For the organic solvent, a general inert solvent such as benzene, toluene, xylene, pyridine can be used. Also, during these reactions, triethylamine, pyridine or other bases may be added in an amount sufficient for the catalyst.

The reactions under the reaction formula C above are carried out in a dichloromethane, chloroform or other organic solvents, in the presence of a desulfurizer, for 30 minutes to 1 hour, at a temperature ranging from 0°C to the boiling point of the solvent, desirably, from 0°C to room temperature. For the desulfurizer,

an ordinary desulfurizer can be used, but it is desirable use tributylphosphine, triphenylphosphine, trimethylphosphite, or other trivalent phosphorus compounds.

Under whichever methods A, B or C the reaction is allowed to go, the reaction is followed by an ordinary after-treatment before the intended substance is obtained.

The structure of the compound of this Invention was determined by means of IR, NMR, MASS, etc.

In the following, Examples are given to explain this Invention in more detail.

Example 1

Synthesis of N-(2,6-difluorobenzoyl)-N'-(2,3-difluoro-4-trifluoromethoxyphenyl) urea (Compound No. 1)

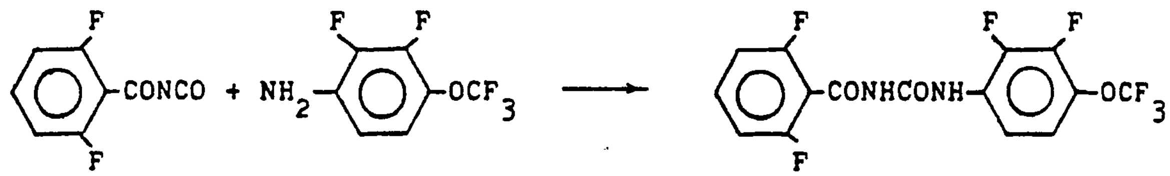

0.50 g of 2,3-difluoro-4-trifluoromethoxyaniline was dissolved in 10 ml of benzene, to which was then added dropwise, under stirring, at room temperature, a solution of 0.45 g of 2,3-difluorobenzoylisocyanate in 5 ml of benzene. Under stirring for 3 hours at room temperature, 80 ml of n-hexane was added to the reaction mixture and the precipitated crystal was filtered and washed with n-hexane. As a result, 0.61 g of N-(2,6-difluorobenzoyl)-N'-(2,3-difluoro-4-trifluoromethoxyphenyl) urea was obtained.

Yield 64%, m.p. 173.5—176°C.

Example 2

Synthesis of N-(2,6-difluorobenzoyl)-N'-(2,3-difluoro-4-trifluoromethoxyphenyl) thiourea (Compound No. 13)

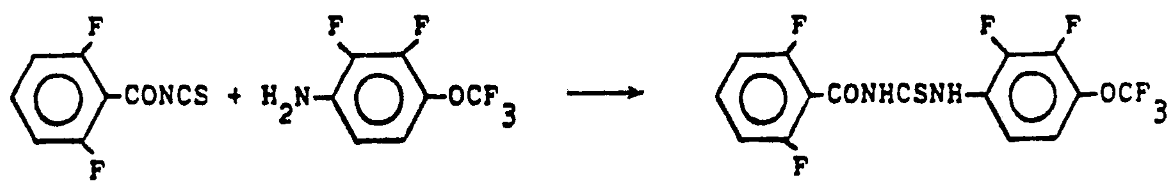

0.90 g of 2,3-difluoro-4-trifluoromethoxyaniline was dissolved in 10 ml of benzene, to which was then added dropwise, under stirring, at room temperature, a solution of 0.90 g of 2,6-difluorobenzoylisocyanate in 5 ml of benzene. Under stirring for 3 hours at room temperature, 80 ml of n-hexane was added to the reaction mixture and the precipitated crystals was filtered and washed with n-hexane. As a result, 1.6 g of N-(2,6-difluorobenzoyl)-N'-(2,3-difluoro-4-trifluoromethoxyphenyl) thiourea was obtained

Yield 83%, m.p. 177—180°C.

Example 3

1-(2,6-difluorothiobenzoyl)-3-(2,3-difluoro-4-trifluoromethoxyphenyl) urea (Compound No. 15)

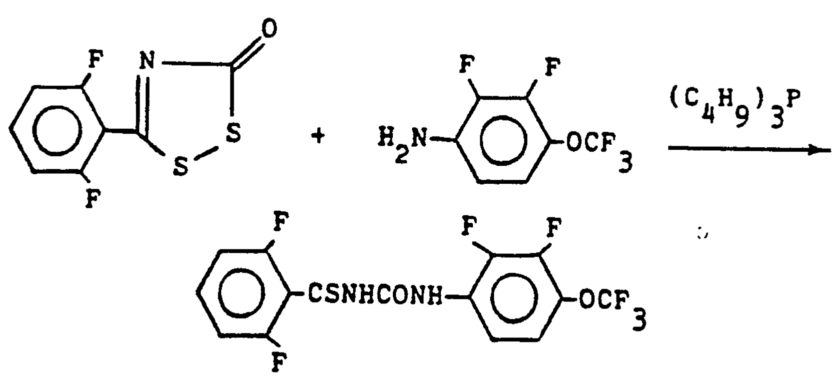


0.43 g of 5-(2,6-difluorophenyl)-1,2,4-dithiazol-3-on was dissolved in 20 ml of dichloromethane. The mixture was cooled to 0°C, to which was then added dropwise, under stirring, a solution of 0.4 g of 2,3-difluoro-4-trifluoromethoxyaniline and 0.38 g of tributylphosphine in 5 ml of dichloromethane.

The reaction mixture was stirred for 30 minutes at room temperature and was then concentrated under reduced pressure. To the concentrate was added n-hexane and as a result the intended substance crystallized out in an amount of 0.45 g.

m.p. 153—154°C.

Table 1 shows typical examples of those compounds of this Invention which were synthesized in the similar method as the above.

TABLE 1

| | Structural formula 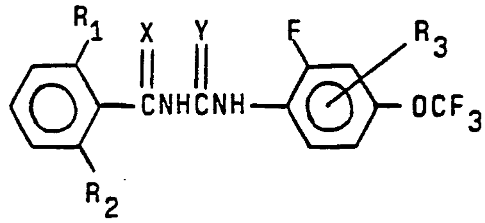 | | | | | |
|---|---|---|---|---|---|---|
| Compound No. | X | Y | $R_1$ | $R_2$ | $R_3$ | Physical Properties m.p. °C |
| 1 | O | O | F | F | 3-F | 173.5~176 |
| 2 | " | " | " | Cl | " | 177~179 |
| 3 | " | " | Cl | " | " | 188~193 |
| 4 | " | " | F | H | " | 139.5~142.5 |
| 5 | " | " | Cl | " | " | 179.5~184 |
| 6 | " | " | Br | " | " | 182~186 |
| 7 | " | " | $CH_3$ | " | " | 192.5~195.5 |
| 8 | " | " | F | F | — ($R_3$=H) | 179~183 |
| 9 | " | " | " | " | 5-F | 179~181 |
| 10 | " | " | " | " | 3-$CH_3$ | 179.5~183.5 |
| 11 | " | " | " | " | 5-$CH_3$ | 180~183 |
| 12 | " | " | " | H | " | 155~160 |
| 13 | " | S | " | F | 3-F | 177~180 |
| 14 | " | " | " | " | — | 148~150 |
| 15 | S | O | " | " | 3-F | 153~154 |
| 16 | " | " | Cl | H | — | 135~139.5 |
| 17 | " | " | F | " | 5-$CH_3$ | 171~173 |
| 18 | " | " | Cl | " | " | 151~153 |
| 19 | " | " | F | F | " | 166~168 |
| 20 | " | " | Cl | H | 5-F | 175~177 |
| 21 | " | " | $CH_3$ | " | " | 179~181 |
| 22 | " | " | F | " | — | 148.5~150 |
| 23 | " | " | " | F | — | 120~124 dec. |
| 24 | " | " | " | " | 5-F | 159~161 |

| Compound No. | Structural formula | | | | | Physical Properties m.p. °C |
|---|---|---|---|---|---|---|
| | X | Y | $R_1$ | $R_2$ | $R_3$ | |
| 25 | S | O | F | H | 5-F | 158~160 |
| 26 | " | " | Cl | " | 3-F | 155~157 dec. |
| 27 | " | " | $CH_3$ | " | " | 145~148 |
| 28 | " | " | F | " | " | 150~153 dec. |
| 29 | O | " | " | F | 5-Cl | 182~184 |
| 30 | S | " | " | " | " | 184~185 |
| 31 | " | " | " | " | 3-$CH_3$ | 163~165 |
| 32 | O | " | $CH_3$ | — | — | 169—170 |
| 33 | " | " | Br | — | — | 118—120 |
| 34 | " | " | Cl | Cl | — | 160—161 |
| 35 | " | " | " | — | — | 115—117 |
| 36 | " | " | F | — | 5-F | 179—181 |
| 37 | " | S | " | F | " | 119—120 |

The insecticides covered by this Invention contain as active ingredients the compounds expressed by the general formula (I). These active ingredients may be used as-produced but normally they are used in any of the forms which ordinary agricultural chemicals can take, namely wettable powder, water soluble powder, dust, emulsifiable concentrate, granule, flowable or other formulations. When solid formulations are intended, then for additives and carriers, soybean flour, wheat flour or other vegetable flour, diatomaceous earth, apatite, gypsum, talc, bentonite, clay, or other fine mineral powders, or sodium benzoate, urea, salt cake or other organic or inorganic compounds are used.

When liquid formulations are intended, then for solvents are used vegetable oil, mineral oil, keronsen, xylene, solvent naphtha or other petroleum fractions, cyclohexane, cyclohexanone, dimethylformamide, dimethylsulfoxide, trichloroethylene, methyl isobutyl ketone, water, etc. A surfactant may, if necessary, be added in order to give a homogeneous and stable formulation. The wettable powder, emulsifiable concentrate, water-soluble powder, flowables, etc., thus obtained are diluted with water to specified concentrations and used as a suspension or an emulsion to spray over the plants. The dust and granule are directly used for spraying over the plants.

Needless to say, the compounds which this Invention covers are sufficiently effective even if they are applied singly. Since these compounds are slow-acting on larvae, however, their application in combination with one or more types of fast-acting insecticides proves to be remarkably effective. Instead of fast-acting insecticides, one or more types of fungicide and/or acaricide can also be used in combination with the compounds of this Invention.

Typical examples of insecticides that can be used together with the compounds of this Invention are as follows.

(Organophosphorus insecticide, carbamate-type insecticide)

fenthion, fenitrotion, diazinon, chlorpyrifos, ESP, vamidothion, phenthoate, dimethoate, formotion, malathon, dipterex, thiomethon, dichlorvos, acephate, cyanophos, pirimiphos-methyl, isoxathion, pyridaphenthion, DMTP, prothiophos, sulprofos, profenofos, CVMP, salithion, EPN, CYP, aldicarb, propoxur, pyrimicarb, methomyl, cartap, carbaryl, thiodicarb, kalphosulfan, carbozulfan, nicotine.

(Pyrethroid-type insecticide)

permethrin, cypermethrin, decamethrin, fenvalerate, fenpropathrin, cyharothrin, flucythrate, fencycrate, tetramethrin, cyfluthriun, fluvalinate, pyrethrin, allethrin, tetramethrin, resmethrin, barthrin, dimethrin, propathrin, prothrin.

Examples of the formulation are given below.

The carrier, surface-active agent, etc., that are added, however, are not limited to these Examples.

Example 4

Emulsifiable concentrate

| | |
|---|---|
| Compound of this Invention | 10 parts |
| Alkylphenyl polyoxyethylene | 5 parts |
| Dimethylformamide | 35 parts |
| Xylene | 50 parts |

These components are mixed and dissolved. For use in spraying, the liquid mixture is diluted with water into an emulsion.

Example 5

Wettable powder

| | |
|---|---|
| Compound of this Invention | 20 parts |
| Higher alcohol sulfuric ester | 5 parts |
| Clay | 74 parts |
| White carbon | 1 part |

These components are mixed and ground to fine powder, which for use in spraying are diluted with water into a suspension.

Example 6

Dust

| | |
|---|---|
| Compound of this Invention | 5 parts |
| Talc | 91 parts |
| Silica | 3 parts |
| Alkylphenyl polyoxyethylene | 1 part |

These are mixed and ground and used in spraying as they are.

Industrial Applicability

In the following, test examples are given to show the insecticidal activity of the compound covered by this Invention.

Test 1

Control effect against tobacco cutworm

In compliance with the formulation of Example 5, compounds of this Invention were each prepared into wettable powders, which were then diluted with water to obtain chemical solutions. In each of these chemical solutions, a leaf of sweet potato was immersed for 30 seconds and air-dried. The treated leaf was put in a petri dish of 9 cm diameter containing 5 tobacco cutworm larvae in the 3rd instar. Covered with the glass lid, the dish was placed in a thermostatic chamber at 25°C and 65% relative humidity. When 5 days elapsed, the mortality was investigated and the 95% lethal concentration ($LC_{95}$) obtained. The results are shown in Table 2.

TABLE 2

| Compound No. | $LC_{95}$ (ppm) |
|---|---|
| 1 | 0.17 |
| 4 | 0.20 |
| 5 | 0.072 |
| 6 | 0.080 |
| 8 | 0.20 |
| 10 | 0.13 |
| 11 | 0.11 |
| 12 | 0.065 |
| 13 | 0.080 |
| 17 | 0.10 |
| Control compound A* | 5.1 |
| Control compound B | 3.2 |
| Control compound C | 4.0 |

*Control compound A :

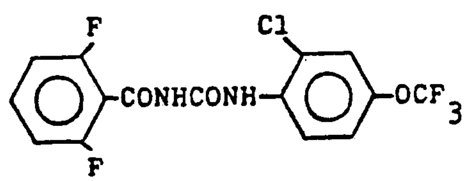

" B :

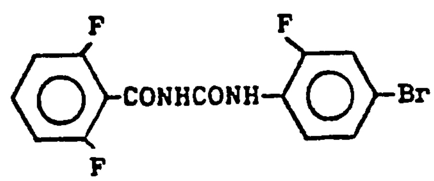

(EP-88343)

" C :

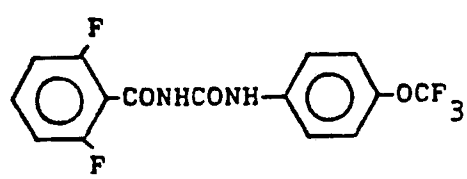

(US-4139636)

Test 2

Control effect against armyworm

In compliance with the formulation of Example 4, compounds of this Invention were each prepared into emulsifiable concentrates, which were then diluted with water to obtain chemical solutions. In each of these chemical solutions, a leaf of corn was immersed for 30 seconds and air-dried. The treated leaf was put in a petri dish of 9 cm diameter containing 5 armyworm larvae in the 3rd instar. Covered with the glass lid, the dish was placed in a thermostatic chamber at 25°C and 65% relative humidity. When 5 days elapsed, the mortality was investigated and the 95% lethal concentration ($LC_{95}$) obtained. The results are shown in Table 3.

TABLE 3

| Compound No. | $LC_{95}$ (ppm) |
| --- | --- |
| 1 | 0.11 |
| 9 | 0.065 |
| 10 | 0.070 |
| 11 | 0.060 |
| 24 | 0.022 |
| Control compound A* | 3.2 |
| Control compound D | 1.7 |

D :

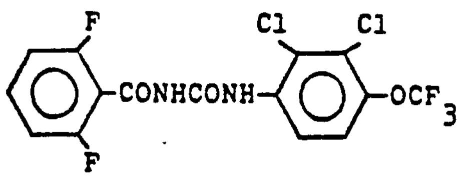

*Control compound A: The same as test 1

* " " D:

Test 3

Control effect against diamond-back moth

In compliance with the formulation of Example 4, compounds of this Invention were each prepared into emulsifiable concentrates, which were then diluted with water to obtain chemical solutions. In each of these chemical solutions, a leaf of cabbage was immersed for 30 seconds and air-dried. Treated leaf was put in a petri dish of 9 cm diameter containing 5 diamondback moth larvae in the 3rd instar. Covered with the glass lid, the dish was placed in a thermostatic chamber at 25°C and 65% relative humidity. When 5 days elapsed, the mortality was investigated and the $LC_{95}$ obtained. The results are shown in Table 4.

TABLE 4

| Compound No. | $LC_{95}$ (ppm) |
| --- | --- |
| 1 | 0.087 |
| 9 | 0.020 |
| 10 | 0.025 |
| 11 | 0.22 |
| Control compound A* | 10.6 |
| Control compound E | >31 |

*Control compound A : The same as test 1

* " E :

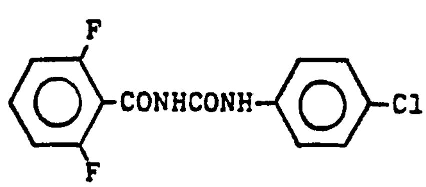

(diflubenzuron)

Test 4

Control effect against tobacco cutworm egg

In compliance with the formulation of Example 4, compounds of this Invention were each diluted with water into concentrations of 500 ppm and 125 ppm. In each of these chemical solutions were immersed for 30 seconds tobacco cutworm eggs, which were then air-dried and placed in a petri dish. Covered with the glass lid, the dish was placed in a thermostatic chamber at 25°C and 65% relative humidity. After 7 days, the mortality was examined and the $LC_{95}$ obtained. The results are shown in Table 5.

TABLE 5

| Compound No. | $LC_{95}$ (ppm) |
|---|---|
| 1 | 0.55 |
| 4 | 0.30 |
| 5 | 0.45 |
| 8 | 0.50 |
| 12 | 0.17 |
| Control compound F* | 12.0 |
| Control compound G | 16.9 |

*Control compound F :

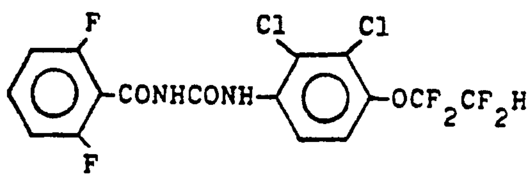

(EP-71279)

" G :

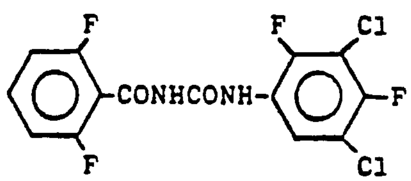

(EP-52833)

**Claims**

1. A compound expressed by the general formula below:

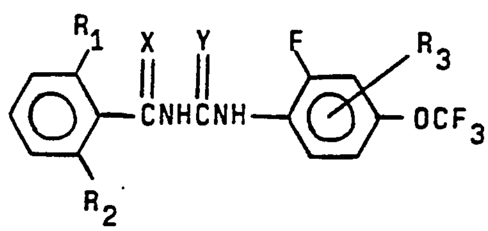

wherein $R_1$ denotes halogen atom or methyl radical, $R_2$ denotes hydrogen atom or halogen atom, $R_3$ denotes hydrogen atom, halogen atom or methyl radical, and each of X and Y denotes oxygen atom or sulfur atom.

2. The compound of claim 1, in which $R_1$, $R_2$ and $R_3$ are fluorine atoms.

3. A process for the production of a compound expressed by the general formula below:

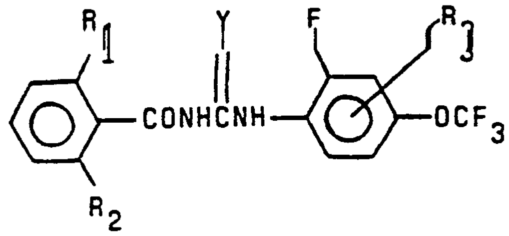

wherein $R_1$ denotes halogen atom or methyl radical, $R_2$ denotes hydrogen atom or halogen atom, $R_3$ denotes hydrogen atom, halogen atom or methyl radical, and Y denotes oxygen atom or sulfur atom, which comprises reacting a compound expressed by the general formula below:

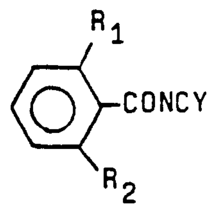

(wherein $R_1$, $R_2$ and Y denote the same as the above) with a compound expressed by the general formula:

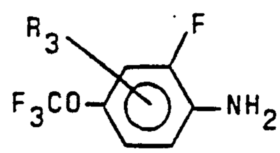

(wherein $R_3$ denotes the same as the above).

4. A process for the production of a compound expressed by the general formula below:

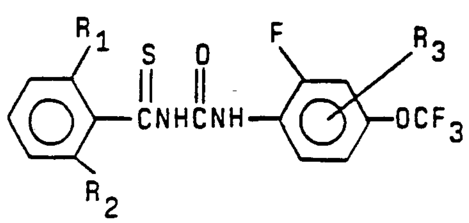

wherein $R_1$ denotes halogen atom or methyl radical, $R_2$ denotes hydrogen atom or halogen atom, $R_3$ denotes hydrogen atom, halogen atom or methyl radical, which comprises reacting a compound expressed by the general formula below:

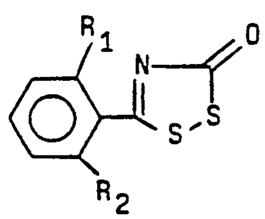

(wherein $R_1$ and $R_2$ denote the same as the above) with a compound expressed by the general formula below:

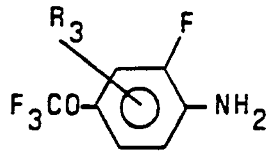

(wherein $R_3$ denotes the same as the above) in the presence of a desulfurizer.

5. The process of claim 4, in which the desulfurizer is a trivalent phosphorus compound.

6. A process for the production of a compound expressed by the general formula below:

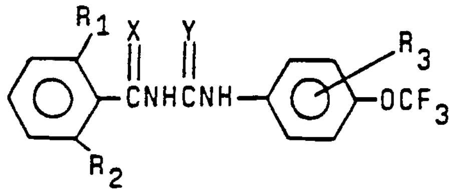

(wherein $R_1$ denotes halogen atom or methyl radical, $R_2$ denotes hydrogen atom or halogen atom, $R_3$ denotes hydrogen atom, halogen atom or methyl radical, and each of X and Y denotes oxygen atom or sulfur atom), which comprises reacting a compound expressed by the general formula below:

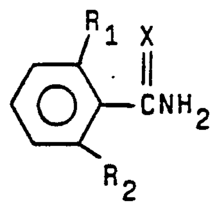

(wherein $R_1$, $R_2$ and X denote the same as the above) with a compound expressed by the general formula below:

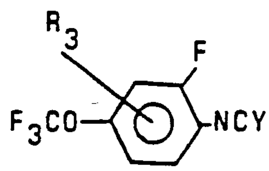

(wherein $R_3$ and Y denote the same as the above).

7. An insecticidal or ovicidal composition containing as active ingredient at least one compound expressed by the general formula below:

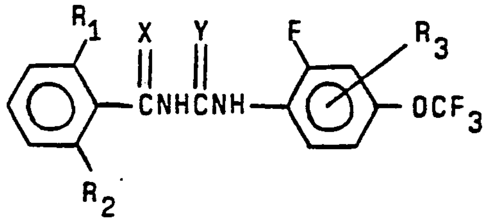

wherein $R_1$ denotes halogen atom or methyl radical, $R_2$ denotes hydrogen atom or halogen atom, $R_3$ denotes hydrogen atom, halogen atom or methyl radical, and each of X and Y denotes oxygen atom or sulfur atom.

8. Use of the compound of claim 1 or 2 for the preparation of pesticidal, in particular insecticidal and/or ovicidal, compositions.

9. Use of the compound of claim 1 or 2 or the composition of claim 7 for controlling pests such as insects and their development stages.

**Patentansprüche**

1. Verbindung, angegeben durch die folgende allgemeine Formel:

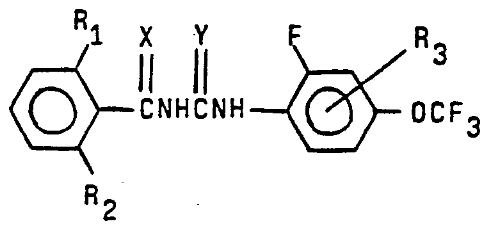

in der $R_1$ ein Halogenatom oder eine Methylgruppe, $R_2$ ein Wasserstoff- oder Halogenatom, $R_3$ ein Wasserstoff- oder Halogenatom oder eine Methylgruppe und X und Y jeweils ein Sauerstoff- oder Schwefelatom bedeuten.

2. Verbindung nach Anspruch 1, in der $R_1$, $R_2$ und $R_3$ Fluoratome sind.

3. Verfahren zur Herstellung einer Verbindung der folgenden allgemeinen Formel:

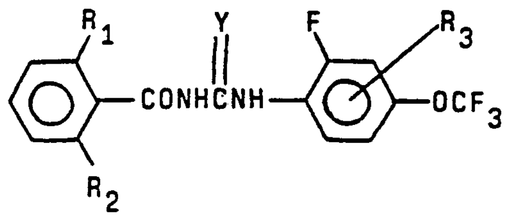

in der $R_1$ ein Halogenatom oder eine Methylgruppe, $R_2$ ein Wasserstoff- oder Halogenatom, $R_3$ ein Wasserstoff- oder Halogenatom oder eine Methylgruppe und Y ein Sauerstoff- oder Schwefelatom bedeutet, umfassend die Umsetzung einer Verbindung, angegeben durch die folgende allgemeine Formel:

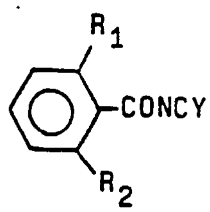

(in der $R_1$, $R_2$ und Y die oben angegebene Bedeutung haben) mit einer Verbindung, angegeben durch die allgemeine folgende Formel

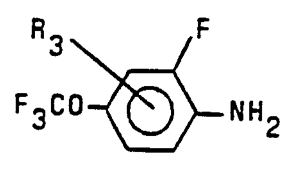

(in der $R_3$ die oben angegebene Bedeutung hat).

4. Verfahren zur Herstellung einer Verbindung, angegeben durch die folgende allgemeine Formel:

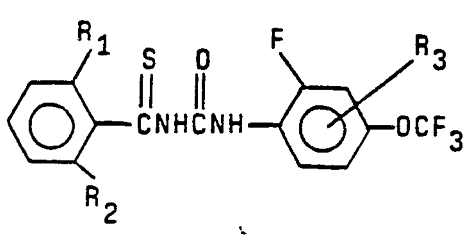

in der $R_1$ ein Halogenatom oder eine Methylgruppe, $R_2$ ein Wasserstoff- oder Halogenatom und $R_3$ ein Wasserstoff- oder Halogenatom eine Methylgruppe bedeutet, umfasend die Umsetzung einer Verbindung,

angegeben durch die folgende allgemeine Formel:

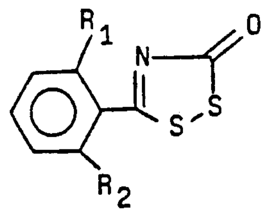

(in der $R_1$ und $R_2$ die oben angegebene Bedeutung haben) mit einer Verbindung, angegeben durch die folgende allgemeine Formel:

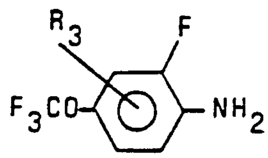

(in der $R_3$ die oben angegebene Bedeutung hat) in Gegenwart eines Entschwefelungsmittels.

5. Verfahren nach Anspruch 4, wobei das Entschwefelungsmittel eine dreiwertige Phorphorverbindung ist.

6. Verfahren zur Herstellung einer Verbindung, angegeben durch die folgende allgemeine Formel:

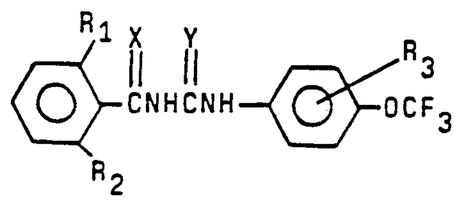

(in der $R_1$ ein Halogenatom oder eine Methylgruppe, $R_2$ ein Wasserstoff- oder Halogenatom, $R_3$ ein Wasserstoff- oder Halogenatom oder eine Methylgruppe und X und Y jeweils ein Sauerstoff- oder Schwefelatom bedeuten) umfassend die Umsetzung einer Verbindung, angegeben durch die folgende allgemeine Formel:

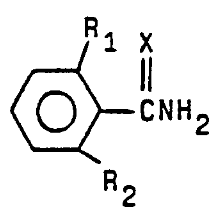

(in der $R_1$, $R_2$ und X die oben angegebene Bedeutung haben) mit einer Verbindung, angegeben durch die folgende allgemeine Formel:

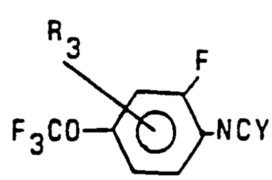

(in der $R_3$ und Y die oben angegebene Bedeutung haben).

7. Insektizides oder ovizides Mittel, enthaltend als aktiven Bestandteil mindestens eine Verbindung, angegeben durch die folgende allgemeine Formel:

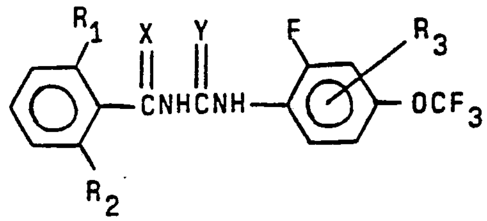

in der $R_1$ ein Halogenatom oder eine Methylgruppe, $R_2$ ein Wasserstoff- oder Halogenatom, $R_3$ ein Wasserstoff- oder Halogenatom oder eine Methylgruppe und X und Y jeweils ein Sauerstoff- oder Schwefelatom bedeuten.

8. Verwendung der Verbindung nach Anspruch 1 oder 2 zur Herstellung von pestiziden, insbesondere insektiziden und/oder oviziden Mitteln.

9. Verwendung der Verbindung nach Anspruch 1 oder 2 oder des Mittels nach Anspruch 7 zur Bekämpfung von Schädlingen wie Insekten und ihren Entwicklungsstadien.

**Revendications**

1. Composé exprimé par la formule générale ci-dessous:

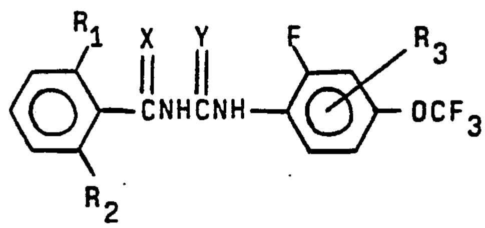

où $R_1$ désigne un atome d'halogène ou un radical méthyle, $R_2$ désigne un atome d'hydrogène ou un atome d'halogène, $R_3$ désigne un atome d'hydrogène, un atome d'halogène ou un radical méthyle et chacun de X et Y désigne un atome d'oxygène ou un atome de soufre.

2. Composé de la revendication 1, dans lequel $R_1$, $R_2$ et $R_3$ sont des atomes de fluor.

3. Procédé pour la production d'un composé exprimé par le formule générale ci-dessous:

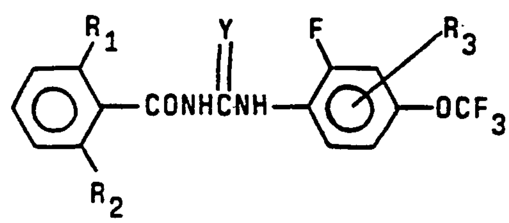

où $R_1$ désigne un atome d'halogène ou un radical méthyle, $R_2$ désigne un atome d'hydrogène ou un atome d'halogène, $R_3$ désigne un atome d'hydrogène, un atome d'halogène ou un radical méthyle et Y désigne un atome d'oxygène ou un atome de soufre, qui comprend la réaction d'un composé exprimé par la formule générale ci-dessous:

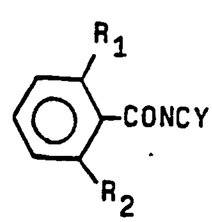

(où $R_1$, $R_2$ et Y désignent la même chose que ci-dessus) avec un composé exprimé par la formule générale:

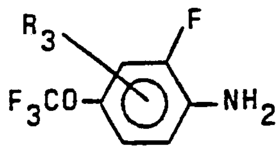

(où $R_3$ désigne la même chose que ci-dessus).

4. Procédé pour la production d'un composé exprimé par la formule générale ci-dessous:

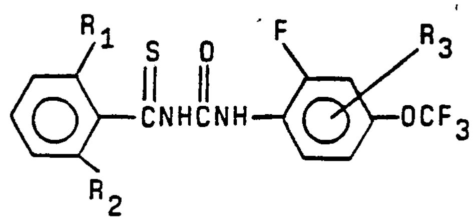

où $R_1$ désigne un atome d'halogène ou un radical méthyle, $R_2$ désigne un atome d'hydrogène ou un atome d'halogène et $R_3$ désigne un atome d'hydrogène, un atome d'halogène ou un radical méthyle, qui comprend la réaction d'un composé exprimé par la formule générale ci-dessous:

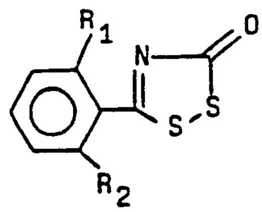

(où $R_1$ et $R_2$ désignent la même chose que ci-dessus) avec un composé exprimé par la formule générale ci-dessous:

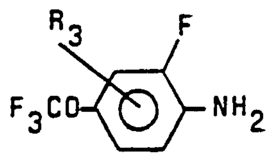

(où $R_3$ désigne la même chose que ci-dessus) en présence d'un agent désulfurant.

5. Procédé de la revendication 4, où l'agent désulfurant est un composé de phosphore trivalent.

6. Procédé pour la production d'un composé exprimé par la formule générale ci-dessous:

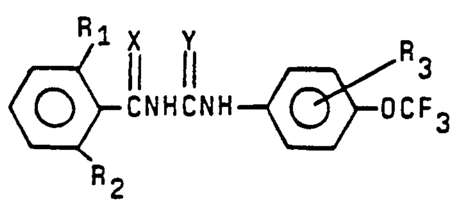

(où $R_1$ désigne un atome d'halogène ou un radical méthyle, $R_2$ désigne un atome d'hydrogène ou un atome d'halogène, $R_3$ désigne un atome d'hydrogène, un atome d'halogène ou un radical méthyle et chacun de X et Y désigne un atome d'oxygène ou un atome de soufre), qui comprend la réaction d'un composé exprimé par la formule générale ci-dessous:

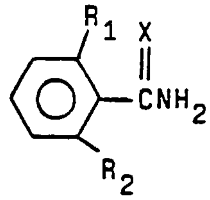

(où $R_1$, $R_2$ et X désignent la même chose que ci-dessus) avec un composé exprimé par la formule générale ci-dessous:

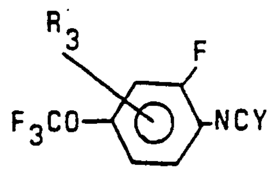

(où $R_3$ et Y désignent la même chose que ci-dessus).

7. Composition insecticide ou ovicide contenant, comme ingrédient actif, au moins un composé exprimé par la formule générale ci-dessous:

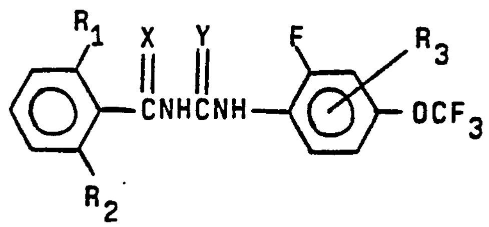

où $R_1$ désigne un atome d'halogène ou un radical méthyle, $R_2$ désigne un atome d'hydrogène ou un atome d'halogène, $R_3$ désigne un atome d'hydrogène, un atome d'halogène ou un radical méthyle et chacun de X et Y désigne un atome d'oxygène ou un atome de soufre.

8. Utilisation du composé de la revendication 1 ou 2 pour la préparation de compositions pesticides, en particulier insecticides et/ou ovicides.

9. Utilisation du composé de la revendication 1 ou 2 ou de la composition de la revendication 7 pour contrôler les pestes telles que les insectes et leur stade de développement.